# EUROPEAN PATENT APPLICATION

(11) **EP 1 097 705 A2**
(43) Date of publication of application: **09.05.2001**
(21) Application number: 00121676.1
(22) Date of filing: 13.10.1997
(51) Int. Cl.: A61K 9/14, A61K 31/015

(54) **Process for the manufacture of a pulverous preparation**

(30) Priority: 14.10.1996 EP 96116420; 25.11.1996 CH 289596
(62) Divisional of application: 97945817.1
(71) Applicant: F. HOFFMANN-LA ROCHE AG, 4070 Basel (CH)
(72) Inventor: Peter, Siegfried, K., F., 91080 Uttenreuth-Weiher (DE); Steiner, Kurt, 4656 Starrkirch-Wil (CH); Stoller, Hansjörg, 4153 Rainach (CH); Weidner, Eckhard, 44795 Bochum (DE)
(74) Representative: Müller, Ingrid, Dr.

(57) **Abstract**

The invention concerns:
A. a process for the manufacture a pulverous active substance selected from carotenoids, especially β-carotene characterized by
   a.1) dissolving the active substance in dimethyl ether under elevated pressure and temperature conditions,
   a-2) flash-decompressing the thus-formed solution in an expansion apparatus, and
   a-3) separating the pulverous solid particles formed in the expansion from the dimethyl ether liberated; and
B. A process for the manufacture of a pulverous preparation which contains an active substance selected from carotenoids, especially β-carotene finally distributed in a matrix component consisting of at least one adjuvant, characterized by
   a) dissolving the active substance under elevated temperature and pressure conditions in a compressed gas in the subcritical or supercritical state,
   b) dispersing the solution obtained from a) in at least one adjuvant,
   c) removing the compressed gas from the dispersion obtained from b),
   d) converting the adjuvant then containing the active substance into a pulverous preparation in a process step known per se.

## Description

The invention is concerned with a process for the manufacture of a pulverous carotenoid finely distributed in a matrix component consisting of at least one adjuvant. The carotenoid is especially β-carotene.

Carotenes are hydrocarbons which, on the basis of numerous conjugated double bonds, often have an intensive colour (mainly red to yellow). Together with oxygen-containing compounds (xanthophylls) they belong to the carotenoid group. These are present in many natural substances, namely as mixture of different carotenoids, e.g. in algae, fungi, vegetable oils, carrots, paprika. Colour concentrates which are used as physiologically harmless colorants in the cosmetic or foodstuff industry can be obtained from certain natural products. Red carotenoids from paprika are used, for example, for the colouring of lipsticks. Disadvantages of these colour concentrates are the different impurities depending on the starting product and the non-uniform composition of the colour-imparting components.

For this reason various syntheses for the production of nature-identical carotenoids especially β-carotenes, have been developed. The preparation of pure carotenes is indispensable primarily with respect to their pharmaceutical application. β-Carotene is of particular interest in this respect. β-Carotene is used as a vitamin A precursor in medicinal preparations. Having regard to its antioxidative activity it is also used in the prophylaxis and therapy of certain cancers. A large number of different synthetic routes for the production of β-carotene have been developed in the past decades. In the industrially used syntheses, e.g. according to Karrer, Pommer, Inhoffen and Isler, the β-carotene obtained in the last step is extracted from the reaction solution with alkanes, e.g. heptane, or chlorinated hydrocarbons, e.g. methylene chloride. After drying there is obtained a solid product which is usually pulverized by milling or dissolved in a conventional solvent, subsequently incorporated into a matrix component and the matrix component containing the thus finely distributed carotenoid is converted into a powder.

A disadvantage is that the solvent concentration in the end product can only be decreased to a value which is harmless to health with the use of considerable technical resources. Furthermore, it is not possible or only very difficult to produce particles having sizes of < 10 µm by milling. However, smaller particle sizes are desirable having regard to bioavailability.

The object of the present invention is to manufacture a pulverous preparation comprising carotenoids, especially β-carotene having a particle size of less than 10 µm, especially less than 1 µm.

Carotenoids, especially β-carotene and a lot of pharmaceuticals are insoluble in water and have only a very low solubility in most organic solvents. This property and the availability in a relatively large particle size stand in the way of a direct use of β-carotene, for example, for the colouring of aqueous foodstuffs or as feed additives or in the cosmetic field. Attempts have therefore been made in the past, and it is one object of the present invention, to manufacture carotenoids, especially β-carotene, having a particle size below 10 µm, especially below 1 µm.

A process in which the β-carotene is dissolved in supercritical fluids, preferably carbon dioxide or dinitrogen monoxide, is proposed in DE-OS 29 43 267. Dinitrogen monoxide has somewhat better dissolving capacity for β-carotene than carbon dioxide. Because of the comparatively poor dissolving properties, pressures up to 500 bar are required in order to obtain a β-carotene concentration of 0.01 wt.% in the supercritical gases. The thus-produced supercritical and highly diluted solutions are rapidly depressurized in a suitable apparatus, e.g. capillaries. The dissolution capacity of the supercritical fluids for carotene is thereby lost and carotene separates in finely divided form. The particle sizes then lie below 1 µm. The separation of these particles from the very large gas stream is achieved by depressurizing the supercritical solution in an aqueous gelatine solution. Thereby, a certain part of the nanometer particles is retained. The gelatine solution, to which is optionally added other adjuvants, e.g. foam preventers, corn starch, glycerine, is dried and subsequently pressed to a solid oral medicament. The process is overall very expensive, since more than 1000 kg of gas are required for the production of 1 kg of pulverous β-carotene.

In an alternative process of Chang and Randolph in Precipitation from Microsize Organic Particles from Supercritical Fluids, AICHE Journal, vol 35, No. 11 (1989): 1876-1882, it is proposed to dissolve β-carotene in supercritical carbon dioxide at a high pressure and elevated temperature. The solubility of β-carotene in carbon dioxide is strongly dependent on pressure and temperature. It increases with increasing pressure and increasing temperature. Solubility of β-carotene in carbon dioxide at 35°C and 500 bar is 0.00049 wt.%. The solubility rises to 0.0016 wt.% in the case of an increase of temperature to 55°C at constant pressure. According to the investigations of Chang and Randolph β-carotene can be crystallized out from the supercritical gas phase by slow pressure and or temperature lowering and the particle sizes, which likewise lie in the nanometer range, and the crystal form can be varied in certain limits by adjusting the speed of the pressure and temperature changes. Disadvantageous in this process are the long dissolution and crystallization times, which lie in the range of 30 min. to several hours. Having regard to the poor solubility, the room-time yields of the process are so low that it is not possible to obtain large amounts of powder in an economical manner.

Jay and Steytler in Nearcritical Fluids as Solvents for β-Carotene, J. of Supercrit. Fluids, 5 (1992): 274-282, investigated the solubility of β-carotene in various supercritical (CO₂, N₂O, C₂H₆, C₂H₄, Xe, SF₆, C₃H₈, CHClF₂, NH₃, CCl₂F₂, SO₂, n-C₄H₁₀) and liquid (n-C₆F₁₄, n-C₅H₁₂, n-C₆H₁₄, n-C₇H₁₆, c-C₆H₁₂, C₂(CH₃)₄, C₆H₆, CCl₄, C₂Cl₄, CS₂, C₂H₅OH, (CH₃)₂CO, CH₂Cl₂) solvents. β-Carotene has only a low solubility in the solvents investigated. The highest concentration of β-carotene in supercritical fluids was measured at 0.035 wt.% for ethylene at 55°C and 500 bar. Dissolution in liquid sulphur dioxide is 0.59 wt.% at 15°C. The concentration of β-carotene lies below 1 wt.% in the solvents which are liquid at room temperature. An exception is CS₂, in which 3.5 wt.% of β-carotene dissolves at room temperature. The disadvantages of the processes described above, which result, inter alia, from the limited solubility of the β-carotene in dinitrogen monoxide and carbon dioxide, can not be overcome by using the other solvents investigated by Jay and Steytler.

A process is proposed in WO 95/21688 in which almost critical or supercritical or generally formulated highly compressible substances are dissolved in organic fluids or form liquid solutions with organic solids which contain in dissolved form the substance to be pulverized. Highly compressible substances which are named are: CO₂, NH₃, N₂O, C₂H₆, C₂H₄, C₃H₈, C₃H₆, CCLF₃, CH₃F, CCl₂F₂, SO₂, n-C₄H₁₀, i-C₄H₁₀, n-C₅H₁₂, as well as at corresponding pressures and temperatures C₂H₅OH, CH₄OH, H₂O, isopropanol, isobutanol, benzene, cyclohexane, cyclohexanol, pyridine, o-xylene. An essential advantage of this mode of operation is that gases have substantially better solubility in organic compounds than vice versa. Typical are values of 5 to 50 wt.% of gas at pressures between 5 and 500 bar, preferably 10 to 200 bar. The gas-containing solutions are rapidly depressurized. The gas is liberated and thereby cools. When a sufficient amount of gas has dissolved out, the cooling can be so strong that the temperature falls below the solidification temperature of the substance to be pulverized. The gas expands strongly during the depressurization. Thereby, the solidified substance disintegrates into very fine particles. In this process between 0.1 kg and 1 kg of gas is required for the production of 1 kg of powder. A further embodiment of this process comprises spraying substances which have a melting lying above the decomposition temperature. In this case it is proposed to add an adjuvant to the substance to be sprayed. The adjuvant is selected such that the mixture of the substance to be pulverized and the adjuvant has a melting point which lies below the decomposition temperature. The highly compressible component is then dissolved in this liquid mixture or solution. A so-called coprecipitate separates with the rapid depressurizing of the gas-containing solution.

In analogy to the procedure described in WO 95/21688 it has been investigated whether β-carotene forms liquid solutions with the mentioned and other highly compressible substances. β-Carotene forms liquid solutions with the gases only at temperatures in the region of its melting point of 180°C. In this temperature region the β-carotene isomerizes or decomposes already after a very short time. It has been found that dimethyl ether is completely miscible with β-carotene at considerably lower temperatures. From PCT Patent Publication WO 96/15133 it is already known that dimethyl ether is an excellent solvent at elevated temperature and pressure conditions. The solubility of β-carotene in liquid dimethyl ether is strongly temperature dependent. About 1.1 wt.% of β-carotene dissolve in liquid dimethyl ether (vapour pressure 4 bar) at 25°C. At 60°C (vapour pressure 15 bar) already 2.7 wt.% dissolve. At higher pressures (up to 300 bar) the solubility is practically not increased in this temperature region. Only at temperatures above 100°C is the solubility of β-carotene in dimethyl ether appreciably pressure dependent. Thus, a homogeneous solution of β-carotene and dimethyl ether exists at 105°C and pressures above 140 bar. In this pressure and temperature region the two substances are miscible without limitation in any ratio.

The objectives posed for the manufacture of a pulverous preparation comprising carotenoids, especially β-carotene, are thus achieved by a process characterized by
a-1) dissolving the carotenoid in dimethyl ether under elevated pressure and temperature conditions,
a-2) flash-decompressing the thus-formed solution in an expansion apparatus, and
a-3) separating the pulverous solid particles formed in the expansion from the dimethyl ether liberated.

Further advantageous developments of the invention are given by the characterizing features of Patent Claims 2 to 10.

Further advantages, features and details of the invention are described by way of example with reference to the drawings
- Figure 1: shows diagrammatically a simple process sequence based on an apparatus usable therefor;
- Figure 2: shows diagrammatically a preferred process sequence based on a preferred embodiment of the apparatus according to Figure 1;
- Figure 3: shows diagrammatically a further preferred embodiment of the apparatus according to Figure 1.

β-Carotene 1 is charged in solid form, preferably having a large surface area, into a suitable high-pressure vessel 10. Dimethyl ether is brought from a reservoir 11 by means of a compressor element 12 to the desired pressure, about 60-500 bar, preferably above 100 bar, preheated in a heat exchanger 13 to about 60-140°C, preferably to 80-140°C, and passed through the β-carotene 1. The β-carotene 1 dissolves in the dimethyl ether. The solution is flash-decompressed using a suitable expansion apparatus 14, e.g. a nozzle, orifice plate, capillary, valve or nozzle/diffuser system. In this simple procedure the final pressure in the expansion is in the order of atmospheric pressure. In a preferred embodiment the expansion device 14 is integrated into a spray tower 15. The gas released is led off or extracted via a line 16. The pulverous solid β-carotene content present therein is separated from the gas stream in a suitable device 17. For this purpose, conventional process engineering equipment such as e.g. cyclones, sieves, fine filters or electrostatic precipitators can be used. The gas 18 released can, if desired, be recovered. During the expansion a finely divided, non-agglomerating β-carotene powder 2 is obtained.

However, it is expedient to decrease the final pressure only to the extent that the complete miscibility between dimethyl ether and β-carotene is eliminated. The β-carotene then precipitates out at elevated pressure as a solid, finely divided, pulverous product 2. The advantage of this variant is the easy recovery of the dimethyl ether.

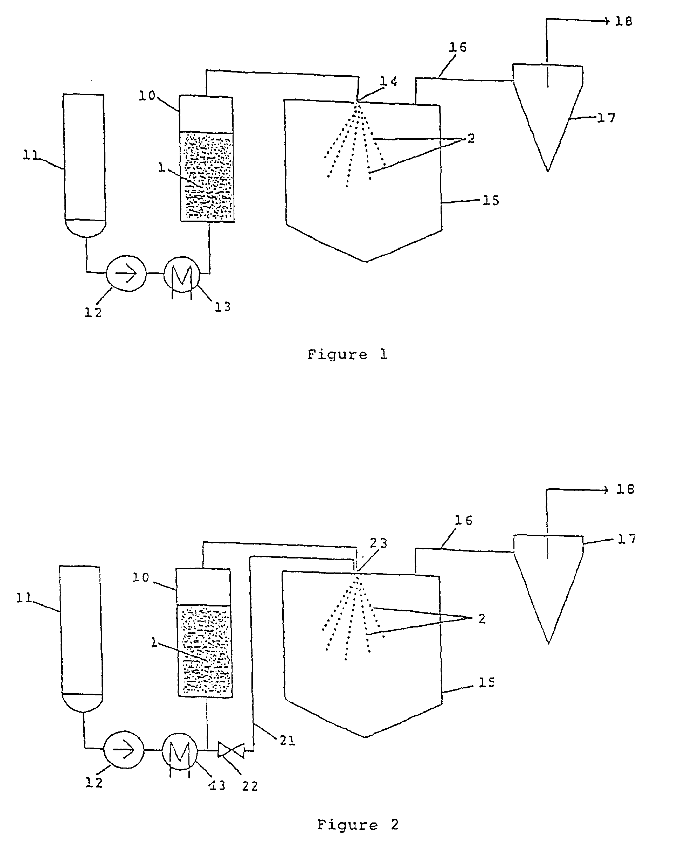

The particle size is below 10 µm, especially below 1 µm. The particle size distribution of the product can be influenced by the selection of the dissolution conditions, the shape of the expansion apparatus 14, in particular the nozzle shape, and other conventional process-engineering measures. In this context, the feed of additional gas during the expansion must be mentioned in particular. This can take place via a separate spray line 20 or in a multi-component expansion apparatus 23 instead of the usual expansion apparatus 14, in particular in a multi-component nozzle. The additional gas used can be dimethyl ether or other gases, preferably liquefied gases, such as carbon dioxide or propane as well as nitrogen. The advantage of using liquefied gases as the additional gas is that intensive cooling usually occurs during their expansion. The supersaturation in the free jet after the expansion may thus be established via the mass stream of the additional gas. Figure 2 shows the preferred apparatus for adding the additional gas. Compressed dimethyl ether is fed in during the expansion of the β-carotene -containing dimethyl ether using a multi-component nozzle 23 via a bypass line 21 having a suitable controllable shut-off apparatus 22.

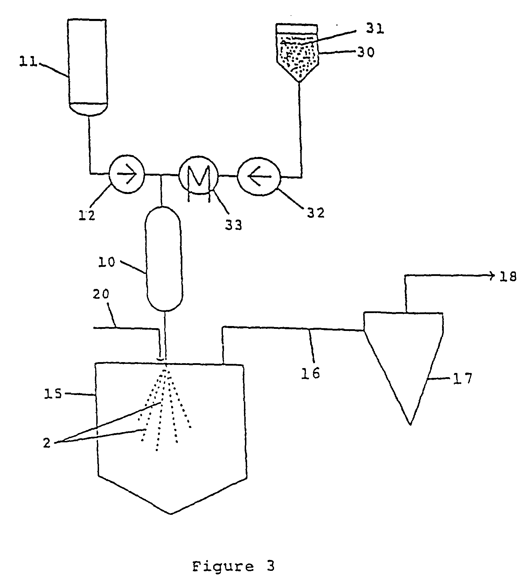

In a further embodiment of the process, which can be seen in Figure 3, an adjuvant is added to the carotenoid 1, especially β-carotene, with which, even at low temperatures, it forms a liquid solution or a pumpable suspension. As the adjuvant use is preferably made of polyethylene glycols of various molecular weights. A mixture 31 of adjuvant and β-carotene is charged into a reservoir tank 30 at temperatures at which the mixture 31 is pumpable. The appropriate temperature and concentration conditions may be varied within wide ranges by suitable choice of the adjuvant. If, for example, polyethylene glycol having a molecular weight of 1500 g/mol is used, the mixtures 31 of polyethylene glycol and β-carotene are pumpable without problem at temperatures from 65°C to a content of 25 wt.% of β-carotene. If polyethylene glycol having a molecular weight of 4000 g/mol is used, at the same β-carotene concentration a temperature of above 80°C is necessary to obtain a pumpable mixture 31. The liquid mixture 31 of β-carotene and adjuvant is then continuously fed to a high-pressure vessel 10 by means of a suitable conveying element 32, downstream of which, if appropriate, a heat exchanger 33 is further connected. Simultaneously, a gas or gas mixture is fed to a high-pressure vessel 10 from a reservoir 11 via a compressor element 12. In the pressure vessel 10, in accordance with the thermodynamic conditions in the mixture of adjuvant and β-carotene, β-carotene, the latter is dissolved and, after the mixture has flowed through the pressure vessel 10, it is expanded. The subsequent procedure is identical to the above-described embodiments. The solution is expanded as already described in an expansion apparatus 15, β-carotene precipitating out as a solid, finely divided pulverous product 2. In a preferred embodiment the gas used is dimethyl ether. Surprisingly, it has now been found that fine powdering of β-carotene from the mixture of polyethylene glycol and β-carotene is also possible using carbon dioxide or preferably using mixtures of carbon dioxide and dimethyl ether, without the disadvantages previously described in connection with carbon dioxide occurring.

Expediently, as pressure vessel 10, use is made of a mixer-autoclave, preferably a static mixer, since in this case only a very small high-pressure volume is necessary. However, the use of other mixer-autoclaves, such as agitators, shakers, pumped-circulation autoclaves, is also possible.

An other embodiment of the invention is to provide a process for the manufacture of a pulverous preparation comprising carotenoids especially β-carotene in which the carotenoid is finely distributed in a matrix component, especially to give rise to a high availability, preferably bioavailability and/or high colour intensity. Preferably, the particle size of the carotenoid should lie below 1 µm, especially between about 0.05 µm and about 0.5 µm.

Starting from the aforementioned state of the art and these findings, the posed object is achieved by the characterizing features of claim 11.

Other advantageous embodiments of the process will be evident from the characterizing features of the subclaims 12-18.

Further advantages and feature of the invention will be evident from the following description of embodiments.

The process for the manufacture of a pulverous preparation comprising carotenoids in which the carotenoid is finely distributed in a matrix component, according to the invention is not only simpler, but also more economical than comparable known processes. In particular, the preparation manufactured according to the process in accordance with the invention using dimethyl ether as the compressed gas is distinguished by the advantage that, because of the high dissolution capacity of dimethyl ether for carotenoids, it has a high content of carotenoid which, moreover, is distributed in the matrix component in the form of very small particles and accordingly owing to its large surface has an excellent availability, especially bioavailability and colour intensity, when the preparation is used.

Possible adjuvants are at least waxes, fats, hydrocolloids, especially starch or a starch derivative such as e.g. maltodextrin, gelatines, plant gums such as gum arabic, polysaccharides, sacharose, proteins of animals such as e.g. lactoprotein, plantprotein and/or proteins of fermentative origin, synthetic polymers, polyvinylpyrrolidone, polylactates or polyacrylates.

For example, the above mentioned properties come to full fruition in the case of a preparation carotenoids, especially β-carotene, and the adjuvant(s) for the matrix component is/are fish gelatine, vegetable proteins or a starch derivative. Thereby, the matrix component acts, inter alia, as a protection for the carotenoid or for its stabilization and is responsible for an optimal resorption and for a water dispersibility of the final preparation which may by required. The β-carotene particles embedded in the matrix component have a size of 0.05-0.5 µm for an optimal availability especially bioavailability and/or high colour intensity. The pulverous preparation has a preferred size of the individual particles of 50-500 µm depending on the purpose of use.

The following Examples describe the invention in more detail.

Examples 1-6 further explain the invention according to claim 1 and referring to a carotenoid, especially β-carotene.

Example 7 describes a process for the manufacture of a pulverous preparation which contains β-carotene finely distributed in a matrix component according to claim 14.

### Example 1

250 g of solid trans-β-carotene were charged into an autoclave having a volume of 1 l. The autoclave was thermostated electrically to a temperature of 105°C. 500 g of dimethyl ether (cosmetic grade) were pumped into the autoclave. Dissolution of the carotene took about 60 minutes, with shaking of the autoclave. The pressure was then 175 x10⁵Pa. A sample was taken at both the top and bottom lids of the autoclave. The sample compositions were determined gravimetrically. Both samples gave a carotene content of 33 wt.% and a dimethyl ether content of 67 wt.%, i.e. a homogeneous phase of carotene and dimethyl ether was present in the autoclave.

The autoclave was connected to a spray tower via a heated high-pressure line, kept at 105°C, and having an internal diameter of 3 mm. A solid-cone nozzle (Schlick, type V121, bore hole 0.3 mm, angle of spray 30°C) was fixed at the end of the high-pressure line below the spray-tower lid. The spray procedure was started by opening a shut-off valve between autoclave and expansion apparatus. Through a viewing port in the spray tower the formation of intensive red solid particles was observed immediately. During the spraying process the pressure in the autoclave was maintained by supplementation with fresh dimethyl ether. The gas liberated in the spray tower was fed to a cyclone together with fines portion of the powder. In the cyclone the carotene was virtually quantitatively separated.

After completion of the spray procedure the spray tower was opened. 230 g of finely particulate carotene powder were taken off. About a further 20 g of powder were present in the cyclone. The concentration of residual solvent resulting from the industrially employed synthesis process was considerably decreased in comparison with the starting material. The residual content of dimethyl ether was still greatly below that of the methylene chloride.

### Example 2

200 g of trans-β-carotene were stirred into 400 g of liquid polyethylene glycol having a molecular weight of 1500 g/mol at a temperature of 75°C. The mixture had roughly the viscosity of honey at this temperature. It was charged in the liquid state into an autoclave having a volume of 1 l. The autoclave was thermostatted electrically to a temperature of 85°C. 350 g of dimethyl ether (cosmetic grade) were pumped into the autoclave. The pressure was 200 x10⁵Pa After a shaking time of 30 min. the spray procedure as described in Example 1 was started. A commercial two-component nozzle having a bore hole diameter of 0.4 mm was used. The solution of polyethylene glycol/β-carotene and dimethyl ether was passed through the inner nozzle. In the annular gap, during the spray procedure, sufficient additional dimethyl ether was added such that the temperature in the spray tower was 25°C. Through a viewing port in the spray tower the formation of intensive red solid particles was observed. During the spray procedure the pressure in the autoclave was maintained by supplementation with fresh dimethyl ether. The gas liberated in the spray tower was fed to a cyclone together with the solids. In the cyclone the solids were separated off virtually quantitatively as very fine powder.

After completion of the spray procedure the spray tower was opened. 550 g of finely divided coprecipitate of polyethylene glycol 1500 and β-carotene were taken off. About a further 112 g of powder were present in the cyclone.

### Example 3

167 g of trans-β-carotene were stirred into 500 g of liquid polyethylene glycol having a molecular weight of 4000 g/mol at a temperature of 90°C. The mixture was charged in the liquid state into an autoclave having a volume of 1 l. The autoclave was thermostatted electrically to a temperature of 80°C. 350 g of dimethyl ether (cosmetic grade) were pumped into the autoclave. After a shaking time of 30 min. the spray procedure was started. The spraying was performed in a similar manner to Example 1. The temperature in the spray tower was set at 40°C. Through a viewing port in the spray tower the formation of intensive red solid particles could be observed immediately. During the spray procedure the pressure in the autoclave was maintained by supplementation with fresh dimethyl ether. The gas liberated in the spray tower was fed to a cyclone together with the fines portion of the powder. After completion of the spray procedure the spray tower was opened. 600 g of finely divided coprecipitate of polyethylene glycol 4000 and β-carotene were taken off. About a further 60 g of powder were present in the cyclone.

### Example 4

167 g of trans-β-carotene were stirred into 500 g of liquid polyethylene glycol having a molecular weight of 1500 g/mol at a temperature of 60°C. The mixture had roughly the viscosity of thin honey at this temperature. It was charged in the liquid state into an autoclave having a volume of 1 l. The autoclave was thermostatted electrically to a temperature of 60°C. Carbon dioxide was pumped into the autoclave to a pressure of 250 x10⁵Pa After shaking time of 30 min. the spray procedure was started. A commercial two-component nozzle having a bore hole diameter of 0.4 mm was used. The solution of polyethylene glycol/β-carotene and carbon dioxide was passed through the inner nozzle. In the annular gap, during the spray procedure sufficient additional carbon dioxide was added such that the temperature in the spray tower was 0°C. Through a viewing port in the spray tower the formation of intensive red solid particles could be observed immediately. During the spray procedure the pressure in the autoclave was maintained by supplementation with fresh carbon dioxide. The gas liberated in the spray tower was fed to a cyclone together with the fines portion of the powder. The solid was separated off in the cyclone virtually quantitatively. After completion of the spray procedure the spray tower was opened. 450 g of finely divided coprecipitate of polyethylene glycol 1500 and β-carotene were taken off. About a further 210 g of powder were present in the cyclone.

### Example 5

2 kg of carotene were stirred into 10 kg of polyethylene glycol having a molecular weight of 1500 g/mol and a temperature of 70°C in a reservoir having a volume of about 20 1. The thin solution was brought to a pressure of 150 x10⁵Pa by means of a metering pump having an output of 8 kg/h. The mixture of polyethylene glycol and β-carotene was mixed with dimethyl ether in a static mixer having an internal diameter of 10 mm and a length of 500 mm. The temperature was 50°C. The total mass flow rate was 20 kg/h. After the mixing procedure the expansion was performed in a spray tower in a nozzle having an internal diameter of 0.5 mm and a spray angle of 120°. 11 kg of a finely divided coprecipitate of polyethylene glycol and β-carotene were obtained in the spray tower. About 1 kg of fines portion were collected in a downstream filter.

### Example 6

250 g of solid trans-apoester (Ethyl-8'-apo-β-carotene-8'oate) were charged into an autoclave. The autoclave was adjusted to a temperature of 67°C. Dimethyl ether (cosmetic grade) were pumped into the autoclave up to a pressure of 150x10⁵Pa and equilibrated for 30 minutes. 1003 g of the solution was sprayed into a spray-tower via a nozzle having a bore hole of 0.2mm. The flow rate was 84g/min. During the spray procedure the pressure in the autoclave was maintained at 150x10⁵Pa by supplementation with fresh dimethyl ether. The sample composition was determined gravimetrically. The content of the apoester in the sprayed solution was 10 wt% . The obtained apoester has a particle size of 9 µm and 96wt% of the apoester was trans apoester.

### Example 7

39.7g of solid apoester containing 97.4% trans -ethyl-8'-apo-β-carotene-8'oate, 10.3g dl-α-tocopherol and 37.9g corn oil were charged into an autoclave equipped with a stirrer and having a volume of 1.4 1. 390g liquid dimethyl ether were added. The pressure was raised to 5x10⁵Pa. Then nitrogen was pumped into the autoclave up to a pressure of 8x10⁵Pa.

A second autoclave connected by a vent tube and having a volume of 2.4 l was charged with a matrix consisting of 124.1g gelatin, 49.7g ascorbylpalmitate sodium salt, 251.7g sucrose and 474.2 g water. Nitrogen was also pumped into the autoclave up to a pressure of 8x10⁵Pa.

Both autoclaves were adjusted to a temperature of 50°C and maintained at 50°C. The pressure was raised to 12x10⁵Pa. Apoester was solubilized completely within 15 minutes under stirring resulting in a dark red solution. This solution containing the dissolved apoester was transferred within 3 minutes through a connecting tube to the second autoclave containing the matrix. During the addition of the apoester solution the matrix was stirred vigorously. After 30 minutes of vigorously stirring dimethylether was slowly evaporated within 35 minutes.

After removing the pressure residual dimethylether as well as 64.3 g of water were removed under vacuum in a thin film evaporator. The particle size of the emulsion was 0.208 µm.

The solution was sprayed using the known starch catch process. A powder containing 4.7% of apoester was obtained. 94.4wt% of the apoester was trans apoester.

## Claims

1. A process for the manufacture a pulverous preparation comprising carotenoids, especially β-carotene, characterized by
a-1) dissolving the carotenoid in dimethyl ether under elevated pressure and temperature conditions,
a-2) flash-decompressing the thus-formed solution in an expansion apparatus, and
a-3) separating the pulverous solid particles formed in the expansion from the dimethyl ether liberated.

2. A process according to claim 1, characterized in that the pressure of step a-1) is between (10 and 500)x10⁵Pa, preferably (60 and 200)x10⁵Pa, especially between (100 and 200) x10⁵Pa.

3. 4. A process according to claim 1 or 2 , characterized in that the temperature of step a-1) is between 40 and 150°C, preferably 80 and 140°C, especially between 100 and 150°C.

4. A process according to any one of claims 1 to 3 , characterized in that the pressure after the expansion lies below the pressure at which there is a complete solution of the carotenoid in the dimethyl ether.

5. A process according to any one of claims 1 to 4 , characterized in that the pressure after the expansion lies below the vapour pressure of the dimethyl ether at the respective expansion temperature.

6. A process according to any one of claims 1 to 5 , characterized in that at least one adjuvant is added to the substance, especially to the carotenoid, under atmospheric conditions so that a liquid pumpable mixture is formed.

7. A process according to claim 6 , characterized in that the adjuvant is polyethylene glycol.

8. A process according to any one of claims 1 to 7 , characterized in that dimethyl ether is partly replaced by carbon dioxide.

9. A process according to any one of claims 6 to 8, characterized in that 1-75 wt.% preferably 5-50 wt.%, especially 10-30 wt.%, of carotenoid are present in the carotenoid mixture.

10. A coprecipitate of polyethylene glycol and a substance which is preferably a carotenoid, especially β-carotene, characterized in that the substance has a particle size of < 10 µm, especially < 1 µm.

11. A process for the manufacture of a pulverous preparation comprising a carotenoid, especially β-carotene finely distributed in a matrix component consisting of at least one adjuvant, characterized by
a) dissolving the carotenoid under elevated temperature and pressure conditions in a compressed gas in the subcritical or supercritical state,
b) dispersing the solution obtained from a) in at least one adjuvant,
c) removing the compressed gas from the dispersion obtained from b),
d) converting the adjuvant then containing the carotenoid into a pulverous preparation in a process step known per se.

12. A process according to claim 11, characterized in that in process step c) the compressed gas is removed from the dispersion by evaporation.

13. A process according to claim 11 or 12 , characterized in that in process step d) the conversion into a pulverous preparation is effected by spray drying.

14. A process according to any one of claims 11 to 13 characterized in that the compressed gas is dimethyl ether.

15. A process according to any one of claims 11 to 14, characterized in that the adjuvant(s) is/are at least one from the group of waxes, fats, hydrocolloids, especially starch or a starch derivative, gelatines, plant gums, polysaccharides, proteins of animal and/or plant and/or fermentative origin, synthetic polymers, polyvinylpyrrolidone, polylactates or polyacrylates.

16. A process according to claim 15 , characterized in that the adjuvant(s) is/are fish gelatine, plant proteins or a starch derivative.

17. A process according to at least one of claims 11 to 16 characterized in that the temperature lies at 50-200°C and the pressure lies at (10-1000)x10⁵Pa, especially at (50-500)xl0⁵Pa.

18. A process according to any one of claims 11 to 17 , characterized in that it is carried out continuously.

19. A pulverous preparation manufactured according to at least one of claims 11 to 18 , characterized in that it consists of a matrix component from at least one adjuvant in which the carotenoid is finely distributed, and the powder has a particles size of about 0.01-3.0 µm especially of 0.05-0.5 µm.

20. A pulverous preparation according to claim 22, characterized in that the carotenoid is β-carotene, preferably with a particle size of about 0.05 µm to 0.5 µm and that the matrix component consists of fish gelatine, plant proteins or a starch derivative.
